(19) Europäisches Patentamt
European Patent Office
Office européen des brevets

(11) Publication number: **0 222 934 A1**

(12) **EUROPEAN PATENT APPLICATION**

(21) Application number: **85306459.0**

(51) Int. Cl.[4]: **C07C 85/06** , C07C 85/18

(22) Date of filing: **11.09.85**

(43) Date of publication of application:
**27.05.87 Bulletin 87/22**

(84) Designated Contracting States:
**DE FR GB IT NL**

(71) Applicant: **THE DOW CHEMICAL COMPANY**
**2030 Dow Center Abbott Road P.O. Box 1967**
**Midland, MI 48640(US)**

(72) Inventor: **Ramirez, Enrique G.**
**60 Southern Oaks Court**
**Lake Jackson Texas 77566(US)**

(74) Representative: **Burford, Anthony Frederick et al**
**W.H. Beck, Greener & Co. 7 Stone Buildings**
**Lincoln's Inn**
**London WC2A 3SZ(GB)**

(54) **Process for preparation of polyalkylenepolyamines.**

(57) The selectivity of a particular polyalkylenepolyamine formed by reacting a vicinal dihaloalkane with ammonia in water in the presence of a strong base is improved by adding to the reaction mixture containing an imine intermediate formed by said reaction a specific polyalkylenepolyamine to obtain in the product a predominant amount of the next higher homologous polyalkylenepolyamine.

EP 0 222 934 A1

# PROCESS FOR PREPARATION OF POLYALKYLENEPOLYAMINES

This invention concerns a novel process for increasing the selectivity of a particular polyalkylenepolyamine by reacting a vicinal dihaloalkane with ammonia in water in the presence of a strong base to form an imine intermediate, then adding a specific polyalkylenepolyamine to obtain in the product a predominant amount of the next higher homologous polyalkylenepolyamine.

The production of ethylenediamine from ethylene dichloride and ammonia is well known. In addition to ethylenediamine, this process results in the formation of higher homologues such as diethylenetriamine, triethylenetetraamine, tetraethylenepentamine and pentaethylenehexamine as well as a small amount of a high-boiling amine residue that cannot be separated into pure components and is not as valuable as the pure compounds.

At times, the demand for the higher homologues exceeds the demand for ethylenediamine, and efforts have been made to increase the relative amounts of the higher components produced. One way in which this can be done is by decreasing the molecular ratio of ammonia to ethylene dichloride employed in the reaction. This method, however, is undesirable since it also results in an increase in the high-boiling residue from the process.

U.S. Patent 2,769,841 describes a process for decreasing the production of diethylenetriamine while increasing the production of the higher homologues. In accordance with this method, diethylene-triamine is recycled and fed to the reactor with the ethylene dichloride and ammonia. This procedure suffers from the disadvantage that the production of undesirable vinyl chloride is increased and the amount of high-boiling residue is increased.

In U.S. Patent 3,372,129 a method is claimed for making high molecular weight polyamines which have substantially no cyclic structures in the product. This process involves the addition of base, e.g. NaOH, to the reaction mixture containing the amine or ammonia and the alkylene dihalide during the last 60-70% of the reaction time in order to maintain the pH of the reaction mixture between about 8.0 and 12.5.

In a related process, the production of alkylenimines is taught in U.S. Patent 3,336,294 and an improvement on that process in U.S. Patent 3,634,396. In the latter patent a process is taught in which a base, e.g. NaOH, is added to the reaction between ammonia and the alkylene dihalide in an incremental manner. This is reported to give a higher yield of the imine than in the former patent.

The present invention is a process for increasing the selectivity to certain of the higher alkylene amines obtained as by-products in the production of an alkylene amine from an alkylene dihalide, e.g. by reacting ethylene dichloride, and ammonia, without a corresponding increase in the amount of high-boiling residue produced and/or amines having a cyclic structure. Certain of the species can be made to predominate in the product. Thus, by feeding one member of the series to the process the next higher member will predominate in the product.

In a process for making higher alkylenepolyamines by reacting a vicinal dihaloalkane with an excess of ammonia in aqueous medium and wherein a strong base is added to the reaction medium during at least a part of the course of the reaction, the improvement comprises:

(1) conducting the reaction at a temperature of from 40°C to 100°C, preferably from 50°C to 70°C;

(2) adding a strong base to the reaction mixture such that the pH is maintained on the basic side;

(3) adding an alkylenepolyamine to said reaction mixture;

(4) heating the reaction mixture to a temperature of from 50°C to 200°C, preferably 100°C to 130°C; and

(5) continuing the reaction to the disappearance of said dihaloalkane, whereby the next higher amine to the amine added is preferentally produced.

The reaction between ammonia and a vicinal dihaloalkane forms an alkyleneimine intermediate product which in turn reacts with ammonia to form a diamine, the diamine can then react with more of the intermediate to form a mixture containing various polyalkylenepolyamines. Preferably, the excess added alkylene polyamine is removed and the desired polyamine products are separated from the product mixture.

It has now been discovered that if the reaction between ammonia and the intermediate alkylenimine is slowed down as taught in U.S. Patent 3,634,396 with a strong base, e.g. NaOH, and then a particular amine added, the product distribution can be changed to make a product in which the higher amines predominate, in particular the next higher one which is a product of the alkylenimine and the amine added as reactant. Thus, for example, if ethylenediamine (EDA) is added to the reaction between ethylenedichloride (EDA) and ammonia ($NH_3$), the predominant species will be diethylenetriamine (DETA).

The dihaloalkane usually is reacted together with ammonia in water at a ratio of ammonia to halogen compound of from 5:1 to 50:1, preferably from 15:1 to 30:1. If the ratio is too low, the selectivity decreases while too high a ratio increases the cost due to the necessity of recycling the excess ammonia.

The ratio of polyamine to haloalkane usually is from 0.25:1 to 20:1, depending upon the amine being reacted. Low ratios cause reduced selectivity to the desired polyamine and high ratios make it necessary to recycle the excess polyamine. Thus, mole ratios of triethylenetetraamine (TETA) to dihaloalkane usually are from 0.25:1 to 5:1, preferably from 1:1 to 2:1. When using ethylene diamine, the ratio usually is from 1:1 to 20:1, preferably from 4:1 to 8:1.

The temperature for reacting the halogen compound with ammonia is from 40°C to 100°C, preferably 50°C to 70°C. When the temperature is too low, the reaction proceeds too slowly, but if too high, selectivity to the desired imine is reduced. The temperature for the reaction of the alkylenepolyamine with the imine is from 50°C to 200°C, preferably 100°C to 130°C. When this temperature is too low, the reaction is too slow, but if too high, the vapor pressure is too high requiring expensive pressure-containing vessels and equipment.

The reaction time is a function of temperature, but generally is from 15 minutes to 10 hours for the imine formation and about the same amount of time for the polyamine reaction.

The pressure employed needs to be sufficient to provide for the reaction to occur in the liquid phase. Generally, this is from 50 to 2,000 psig (350 to 14,000 kPa) and preferably from about 200 to 600 psig - (1,400 to 4,100 kPa).

The strong base added to neutralize the amine hydrochloride is preferably sodium hydroxide, but other bases such as potassium hydroxide and calcium hydroxide can be used effectively. Alternatively the oxides of the metals can be employed.

Preferably the strong base should be added in an intermittent or continuous manner during at least a part of the course of the reaction of ammonia with the halogen compound. The rate of addition is controlled by the rate at which this reaction proceeds, which in turn is a function of temperature. The pH of this reaction should be kept on the basic side to assure formation of the imine intermediate but if too much base is added, the undesirable dehydrohalogenation product, e.g. vinyl chloride, will be formed.

While 1,2-dichloroethane is exemplified other vicinal dihaloalkanes can be employed. Thus, for example, 1,2-dichloropropane and other vicinal dihaloalkanes such as 1,2-dibromopropane, 2,3-dibromobutane, 1,2-dichlorobutane, 1,2-dichloropentane, 2,3-dibromopentane, 1,2-dichloro-2-methylpropane and the like vicinal halogen containing alkanes containing from 2 to 6 carbon atoms can be employed.

With respect to the polyamine reactant, alkylenepolyamines having the formula

$$H_2N\left(CH_2\underset{\displaystyle R}{\underset{|}{C}}HNH\right)_nH$$

wherein R is hydrogen, methyl or ethyl and n is an integer of from 1 to 4, can be used in the reaction to form a product mixture in which the predominant species is the polyamine wherein n is the next higher integer. Thus, ethylenediamine, diethylenetriamine, triethylenetetramine, tetraethylenepentamine (TEPA) and the propylene and butylene analogous polyamines are useful. For example, when diethylenetriamine (DETA) is added to the reaction, triethylenetetraamine (TETA) will be the pre dominant species, and when triethylenetetraamine (TETA) is added tetraethylenepentaamine (TEPA) will predominate. Unexpectedly, this process makes less of the cyclic products than the previously known processes.

The following examples are representative both of the prior art (comparative) and of the present invention:

## Examples

A two-liter stainless steel autoclave reactor was set up with heating, cooling and agitating capabilities.

Comparative Example A

The reactor was charged with 340 grams of water at ambient temperature. Twenty mole (3400 g) of ammonia were added. The agitator was started and the mixture heated to 130°C. One mole of ethylenedichloride (EDC) was added. The mixture was reacted until complete EDC conversion (60 minutes).

Comparative Example B

The procedure in Example A above was employed except that 272 grams of water, 272 grams (16 moles) of ammonia and 198 grams (2 moles) of EDC were reacted.

To analyze the product, excess ammonia was flashed carefully at 40°C. Fifty percent NaOH was added slowly to the reaction product. Then NaOH pellets were added until an amine layer was formed. The amine layer was analyzed by a gas chromatograph. Results are shown in the table following the examples.

Example 1

The reactor was charged (at ambient temperature) with 0.5 moles (49.5 g) ethylene dichloride (EDC), 10 moles (170 g) ammonia and 5.16 moles (93 g) of water. The agitator was started and the mixture was rapidly warmed up to 55°C (12 minutes). The mixture was allowed to react for 1½ hours at 55°C. Thirty percent aqueous NaOH was then pumped into the reaction mixture at a rate of 0.75 milliliters per minute. A total of 107 grams of 30 wt. percent NaOH (0.8 mole) was added. After the NaOH was added, 0.75 mole (109.5 g) of triethylenetetramine (TETA) was added to the reaction mixture all at once. The reaction mixture was then heated to 110°C and allowed to react for 2 hours until all of the EDC had reacted. The reaction mixture was then cooled and analyzed by gas chromatography.

The product analysis showed 0.65 mole (95 g) of TETA, 0.1 mole (19 g) tetraethylenepentamine (TEPA), and 0.14 mole (8.4 g) of ethylenediamine (EDA). Other amines produced were diethylenetriamine (DETA), piperazine (PIP), aminoethylpiperazine (AEP), vinyl chloride, and polyamines with molecular weights greater than TEPA.

Example 2

The procedure of Example 1 was repeated except that 1.0 mole (146 g) of TETA was used instead of 0.75 mole (109.5 g). The product analysis showed 0.11 mole (20.8 g) TEPA, 0.828 mole (127 g) TETA and 0.13 mole (7.8 g) EDA.

Example 3

The procedure of Example 1 was repeated except that 3.0 moles (180 g) EDA was used instead of 0.75 mole (109.5 g) TETA. The product analysis showed 0.13 mole (13.4 g) DETA, 0.041 mole (6 g) TETA and 2.56 moles (153.6 g) EDA. Vinyl chloride selectivity was 7.2%. All of the EDC was reacted.

Example 4

The procedure of Example 2 was repeated but approximately 10 moles (170 g) of ammonia were flashed after the NaOH addition and before the TETA addition. All other conditions remained essentially the same. The product analysis showed 0.1215 mole (23 g) TEPA, 0.794 mole (116 g) TETA, and 0.073 mole (4.9 g) EDA.

After the product was separated from the excess reactant polyamine the remaining product has the analysis shown in the following table. The product distribution normally found when employing the method of the prior art is shown also as a comparative example, indicated as Example A. The mole ratio of $NH_3$/EDC was 20:1, the same in all examples including comparative Examples A. Another experiment, comparative Example B, was run at an $NH_3$/EDC ratio of 8:1.

TABLE

| Formed Product wt % | Example Number A | B | 1 | 2 | 3 | 4 |
|---|---|---|---|---|---|---|
| EDA | 55.3 | 42.5 | 26.1 | 23.0 | - | 13.5 |
| PIP | 1.9 | 1.4 | 1.3 | 0.6 | 2.9 | 0.5 |
| DETA | 23.3 | 24.1 | 4.4 | 1.4 | 67.0 | 1.9 |
| AEP | 3.5 | 2.8 | 0.7 | 0.6 | 0.3 | 0.9 |
| TETA | 9.9 | 13.9 | - | - | 29.8 | - |
| TEPA | 3.9 | 7.7 | 56.4 | 63.3 | - | 71.0 |
| HIGHERS | 2.3 | 7.6 | 11.1 | 11.0 | - | 12.3 |
| EDA/TEPA By wt | 14.55 | 5.52 | 0.46 | 0.36 | - | 0.19 |

## Claims

1. A process for making higher alkylenepolyamines by reacting a vicinal dihaloalkane with an excess of ammonia in aqueous medium and wherein a strong base is added to the reaction medium during at least a part of the course of the reaction, characterised in that:

(1) the reaction of the dihaloalkane with ammonia is conducted at a temperature of from 40°C to 100°C;

(2) a strong base is added to the reaction mixture such that the pH is maintained on the basic side;

(3) an alkylenepolyamine is added to said reaction mixture;

(4) the reaction mixture containing the alkylenepolyamine is heated to a temperature of from 50°C to 200°C; and

(5) the reaction is continued to the disappearance of said dihaloalkane, whereby the next higher amine to the amine added is preferentially produced.

2. A process as claimed in Claim 1, wherein the reaction of the dihaloalkane with ammonia is conducted at 50°C to 70°C and the reaction mixture containing the alkylenepolyamine is heated to 100°C to 130°C.

3. A process as claimed in Claim 1 or Claim 2, wherein excess added alkylenepolyamine is removed.

4. A process as claimed in any one of the preceding claims, wherein the desired polyamine products are separated from the product mixture.

5. A process as claimed in any one of the preceding claims, wherein the strong base is added in an incremental or continuous manner.

6. A process as claimed in any one of the preceding claims, wherein the mole ratio of ammonia to vicinal dihaloalkane is from 5:1 to 50:1.

7. A process as claimed in any one of the preceding claims, wherein the mole ratio of polyamine to vicinal dihaloalkane is from 0.25:1 to 20:1.

8. A process as claimed in any one of the preceding claims, wherein the alkylenepolyamine has the formula

$$H_2N\left(CH_2\underset{\underset{R}{|}}{CH}NH\right)_nH$$

wherein R is hydrogen, methyl or ethyl and n is an integer of from 1 to 4.

9. A process as claimed in Claim 8, wherein R is hydrogen and the vicinal dihaloalkane is 1,2-dichloroethane.

10. A process as claimed in Claim 9, wherein n is 1, 2 or 3.

European Patent Office

# EUROPEAN SEARCH REPORT

Application number

EP 85 30 6459

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 075 941 (UNION CARBIDE CORP.) * Page 1, lines 12-19; page 12; page 15, line 29 - page 16, line 21; page 37 - page 43, line 24 * --- | 1-10 | C 07 C 85/06<br>C 07 C 85/18 |
| A | US-A-3 761 522 (J.G. SCHNEIDER) * Column 1, lines 34-65 * --- | 1-10 | |
| D,A | US-A-3 634 396 (E.G. RAMIREZ) * Examples * --- | 1-10 | |
| D,A | US-A-2 769 841 (S.W. DYLEWSKI) * Columns 3,4 * ----- | 1-10 | |
| | | | TECHNICAL FIELDS SEARCHED (Int. Cl.4) |
| | | | C 07 C 85/00 |

The present search report has been drawn up for all claims

| Place of search | Date of completion of the search | Examiner |
|---|---|---|
| THE HAGUE | 13-05-1986 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82